# EUROPÄISCHE PATENTSCHRIFT

(11) **EP 0 668 258 B1**
(45) Veröffentlichungstag und Bekanntmachung des Hinweises auf die Patenterteilung: **15.04.1998**
(21) Anmeldenummer: 94119209.8
(22) Anmeldetag: 06.12.1994
(51) Int. Cl.: C07C 45/45, C07C 49/293

(54) **Verfahren zur Herstellung von 1-Cyclopropylalkan-1,3-dionen**
Process for the preparation of 1-cyclopropylalkane-1,3-diones
Procédé pour la préparation de 1-cyclopropylalkane-1,3-diones

(30) Priorität: 09.02.1994 DE 4404059
(43) Veröffentlichungstag der Anmeldung: 23.08.1995
(73) Patentinhaber: HÜLS AKTIENGESELLSCHAFT, 45764 Marl (DE)
(72) Erfinder: Muhr, Dr. Jürgen, D-53347 Alfter (DE)

(56) Entgegenhaltungen:
- EP-A- 0 410 726
- EP-A- 0 454 624
- JOURNAL OF ORGANIC CHEMISTRY, Bd. 17, 1952 EASTON US, Seiten 685-692, G.W. CANNON ET AL. 'Acylation Studies. I. Methyl cyclopropyl Ketone'

## Beschreibung

Die Erfindung beschreibt ein Verfahren zur Darstellung von 1-Cyclopropylalkan-1,3-dionen aus Alkalialkoholat, einem Carbonsäureester mit mindestens einem Wasserstoffatom am α-Kohlenstoff des Carbonsäureskelettes und Cyclopropylalkylketonen.

Cyclopropylalkan-1,3-dione sind Vorprodukte für zahlreiche Pestizide.

Die Darstellung der Cyclopropylalkan-1,3-dione aus Cyclopropylalkylketonen und Carbonsäurederivaten ist in der Literatur bekannt, wobei in der Mehrzahl reaktivste Carbonsäurespezies, wie etwa Essigsäureanhydrid oder Essigsäurechlorid, mit zum Beispiel Cyclopropylmethylketon (CPMK) zur Reaktion gebracht werden und die Umsetzung in Gegenwart von aggressiven Katalysatoren oder Hilfsstoffen, z. B. BF₃, durchgeführt wird (US 3 507 958).

Die Verwendung von Acetylchlorid oder BF₃ bei industriellen Prozessen ist mit einem besonders hohen technischen Aufwand aufgrund der hochtoxischen und stark korrodierenden Eigenschaften dieser hydrolyseempfindlichen Chemikalien sowie deren Zerfallsprodukte verbunden.

Bei deutlich gesenktem Gefahrenpotential und vereinfachtem Handling bieten sich Carbonsäureester, wie zum Beispiel Essigester, als Synthesepartner des Cyclopropylalkylketons, beispielsweise des CPMK, an. Bislang wurden entsprechende Kondensationen allerdings unter Verwendung stärkster Basen, wie z. B. Natriumhydrid (US 3 507 958) oder Natriumamid (Cannon u. Widden, J. Org. Chem. 17, 685 (1952)), bei relativ unbefriedigenden Ausbeuten von rund 40 bis 75 % d. Th. realisiert.

Natriumamid und -hydrid bergen naturgemäß erhebliche Risiken beim Einsatz, da sich in Gegenwart von Feuchtigkeit, Luft und Kohlendioxid explosible Produkte bilden und daher umfangreiche Sicherheitsmaßnahmen notwendig machen.

Die mit Natriumhydrid durchgeführten Synthesen sind des weiteren durch unwirtschaftliche Raum-Zeit-Ausbeuten mit Reaktionszeiten von bis zu 14 Stunden geprägt.

Ein Verzicht auf derart kräftige Basen und Ersatz durch beispielsweise weniger basische Alkalialkoholate niederer Alkohole, wie z. B. Natrium(m)ethylat, erscheint nach EP-A-0 410 726 nicht sinnvoll. Hiernach erzielt man durch Reaktion von CPMK und Essigester mit Natriumethylat lediglich eine Ausbeute von ca. 21 % d. Th. und eine 75 %ige Qualität, selbst dann, wenn die Kondensation bei hohen Temperaturen durchgeführt, der Alkohol zur Verschiebung des Gleichgewichts laufend aus dem Reaktionsgemisch abdestilliert und zusätzlich der Essigester in großem Überschuß eingesetzt wird. Zur Abtrennung der Nebenprodukte ist ein für eine industrielle Fertigung aufwendiger Filtrationsschritt erforderlich, der sich bei einer 75 %igen Reinheit nicht wirksam niederschlägt und für eine Vermarktung zusätzliche Aufarbeitungsschritte notwendig macht. Die unbefriedigenden Ausbeuten sind nicht nur unter dem ökonomischen Aspekt problematisch, sondern müssen auch unter ökologischen Gesichtspunkten kritisch betrachtet werden, da die Entsorgung größerer Mengen an Nebenprodukten notwendig wird.

EP-A-0 454 624 beschreibt ein Verfahren zur Herstellung von 1,3-Diketonen. In diesem Dokument wird gelehrt, daß, um hohe Ausbeuten zu erhalten, die Reaktion bei Temperaturen unter 50°C und in Gegenwart von weniger als 5%, insbesondere weniger als 1% Alkohol oder sogar ohne Alkohol, durchgeführt werden soll.

Aufgabe war es, ein Verfahren zu entwickeln, welches die genannten Nachteile vermeidet und 1-Cyclopropylalkan-1,3-dione mit einfachen Basen in hoher Ausbeute liefert.

Die Aufgabe wird erfindungsgemäß dadurch gelöst, daß man bei der Umsetzung von Carbonsäureestern der Formel I mit Alkoholaten der Formel II und Cyclopropylketonen der Formel III zu den 1-Cyclopropylalkan-1,3-dionen der Formel IV nach dem Schema wobei 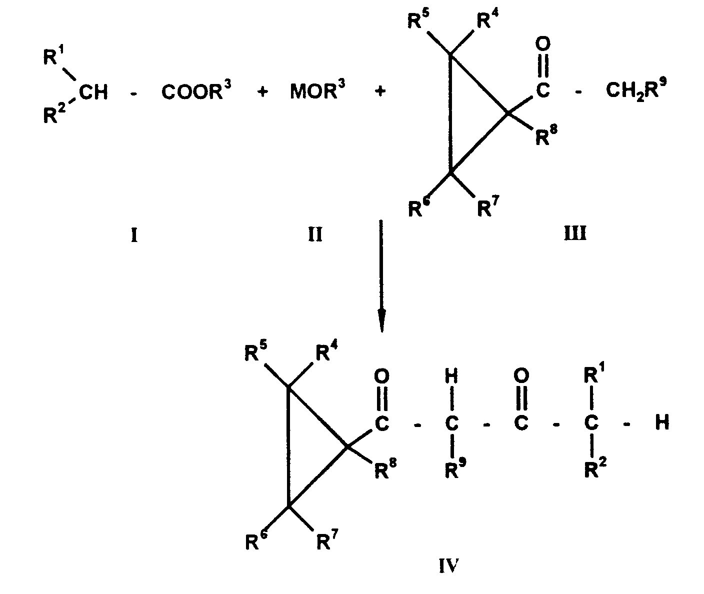
R¹, R² sowie R⁴ bis R⁸ gleiche oder unterschiedliche Bedeutung haben und für Wasserstoff, Alkyl mit 1 bis 6 C-Atomen oder für einen ggf. substituierten Arylrest mit 6 bis 10 C-Atomen steht,
R³ eine Alkylgruppe mit 1 bis 10 C-Atomen ist,
M ein Alkalimetall bedeutet und
R⁹ Wasserstoff oder Alkyl mit 1 bis 20 C-Atomen sein kann, die Reaktion, bei der der Carbonsäureester (Formel I) nicht vor dem Cyclopropylketon (Formel III) mit dem Alkalialkoholat (Formel II) in Kontakt tritt, bei Temperaturen unterhalb 50 °C in einem Lösemittel, das alkoholfrei ist oder bis zu 0,7 mol Alkohol pro mol Alkoholat enthält, durchführt.

Aufgrund der im Vergleich zu Alkalihydriden und -amiden geringeren Reaktivität der Alkalialkoholate sollte sich die gewünschte Kondensationsreaktion erst bei höheren Temperaturen einstellen, was aus EP-A-0 410 726 abgeleitet werden kann. Wider Erwarten gelingt eine Synthese mit Alkalialkoholaten bei deutlich niedrigeren Temperaturen, vorzugsweise im Bereich zwischen -20 und 50 °C, in hohen Ausbeuten ohne Maßnahmen zur Verschiebung des Reaktionsgleichgewichtes durch Einspeisung von überschüssigen Edukten und Abtrennung von Reaktionsalkohol.

Die Substituenten R¹, R² und R⁴ bis R⁸ können als Alkylreste beispielsweise Methyl, Ethyl, Propyl, Isopropyl, n-Butyl, Isobutyl oder Hexyl sein. Die Alkylgruppen können verzweigt oder geradkettig, cyclisch, gesättigt oder olefinisch ungesättigt sein. Es können auch je 2 der Substituenten zu einem ggf. substituierten Cycloalkyl miteinander verbunden sein. Vorzugsweise steht R¹ und R² für Wasserstoff.

Beispiele für R³ sind Methyl, Ethyl, Isopropyl, Butyl, Hexyl und Octyl. Vorzugsweise steht R³ für ein Alkyl mit 1 bis 6 C-Atomen, im besonderen für Methyl oder Ethyl.

Als Alkalimetalle kommen Lithium, Natrium, Kalium, Rubidium und Cäsium in Betracht. Natrium und Kalium werden dabei bevorzugt.

R⁹ kann neben Wasserstoff beispielsweise Methyl, Ethyl, Butyl, t-Butyl, Ethylhexyl oder Dodecyl sein. Allgemein kann der Alkylrest gesättigt oder olefinisch, verzweigt, geradkettig oder cyclisch sein. Er kann ggf. auch mit einkernigem Aryl oder Alkoxy substituiert sein.

In einer bevorzugten Ausführung der Erfindung stehen R⁴ bis R⁹ für Wasserstoff.

Die Acylierung der Cyclopropylketone mit den Carbonsäureestern erfolgt besonders bevorzugt bei Temperaturen von 0 bis 40 °C.

Bezogen auf 1 mol Cyclopropylketon werden vorzugsweise 1 bis 5 mol, besonders bevorzugt 1 bis 2 mol Alkalialkoholat und 1 bis 9 mol, vorzugsweise 1 bis 2 mol Carbonsäureester zur Synthese eingebracht.

Als Alkalialkoholat werden im besonderen das Methylat und das Ethylat von Kalium und Natrium verwendet.

Erfindungsgemäß wird die Reaktion in Gegenwart eines Lösemittels durchgeführt, das keine oder nur geringfügige Mengen an Alkoholen enthält. Bevorzugt sind mit Wasser wenig mischbare Solventien, die bei der Aufarbeitung als Extraktionsmittel verwendet werden können. Grundsätzlich kommen alle gegenüber den Edukten inerte Solventien in Frage, wie zum Beispiel aromatische, aliphatische und/oder araliphatische Kohlenwasserstoffe, aromatische Halogenkohlenwasserstoffe, Ether oder Polyether, umesterungs-bzw. verseifungsstabile Ester, Amide und Amine. Sehr bevorzugt sind Ether, besonders tert.-Butylmethylether, einzusetzen.

Vor dem Hintergrund einer vereinfachten Aufarbeitung kann bei dem dieser Erfindung zugrundeliegenden Verfahren auf Fremdlösungsmittel verzichtet werden, indem man die Reaktion in überschüssigen Edukten als Solvens, vorzugsweise mit dem zur Acylierung verwendeten Ester, durchführt.

Alkohol ist als Reaktionsmedium oder als Zusatz in Mengen, wie sie beispielsweise bei der Verwendung handelsüblicher 20 bis 30 %iger Alkoholatlösungen eingebracht würden, nicht geeignet. Alkohol in diesen Größenordnungen inhibiert die Reaktion. Geringe Zusätze bis zu 0,7 mol eines Alkohols pro mol Alkalialkoholat sind als Additiv zulässig, ohne die Ausbeute wesentlich zu erniedrigen.
Derartige Zusätze können sich positiv hinsichtlich der rheologischen Eigenschaften des Reaktionsgemisches auswirken. Speziell für den technischen Maßstab kann dies eine Steigerung der Raum-Zeit-Ausbeute durch konzentriertere Fahrweise und ein minimiertes Betriebsrisiko durch eine effektivere Wärmeverteilung bedeuten. Als Additive eignen sich grundsätzlich verzweigte und/oder geradkettige aliphatische und araliphatische Alkohole, wie z. B. tert.-Butanol, Polyethylenglykole, deren Ether, insbesondere Diethylenglykoldimethylether.

Vorzugsweise wird 1-Cyclopropylbutan-1,3-dion hergestellt, wobei gemäß Formelschema R¹, R² sowie R⁴ bis R⁹ Wasserstoff bedeuten. Das Produkt wird dann durch Acylierung von CPMK mit einem Essigsäureester, vorzugsweise dem Methyl- oder Ethylester, und mit vorzugsweise Natrium- oder Kaliumalkoholat, im besonderen mit dem Methylat oder Ethylat, gewonnen.

Im Falle der Acylierung des CPMK mit Essigsäureestern kann auf ein Abdestillieren des entstandenen Alkoholes verzichtet werden, da bei einer solchen Vorgehensweise zwangsläufig eine Abreicherung der niedrigsiedenden Esterkomponente erfolgt und daher die Ausbeute minimiert würde. Zudem erfordert eine Destillation im beanspruchten Temperaturbereich einen erheblichen apparativen Mehraufwand, da diese zum einen im Vakuum bei schlechter Trennleistung durchzuführen wäre. Zum zweiten bereitet die Rückgewinnung des unbeabsichtigt abdestillierten Esters zusätzlichen Aufwand und Kosten.

Das Verfahren kann sowohl kontinuierlich, beispielsweise im Strömungsrohrreaktor, als auch batch-weise im diskontinuierlichen Rührkessel praktiziert werden.

Die 1-Cyclopropylalkan-1,3-dione fallen bei dem hier beschriebenen neuen Verfahren zunächst als Enolat an und können in dieser Form, z. B. per Filtration oder nach Abdestillieren flüchtiger Bestandteile, gewonnen und daraus nach bekannten Methoden durch Ansäuern in Freiheit gesetzt werden.

Bei der vorliegenden Erfindung wird jedoch vorzugsweise ohne Isolierung des Salzes aufgearbeitet, indem man das Diketon durch Ansäuern des Reaktionsgemisches freisetzt und nach üblichen Methoden, z. B. per Extraktion oder Destillation, isoliert. Im Falle einer Isolierung via Extraktion kann mit dem schon bei der Reaktion als Medium eingesetzten Solvens extrahiert werden.

Bei der praktischen Durchführung des erfindungsgemäßen Verfahrens wird vorzugsweise dafür gesorgt, daß die Esterkomponente zu den ggf. in einem der genannten Lösemitteln ganz oder teilweise vorgelegten Keton- und Alkoholatkomponenten portionsweise oder kontinuierlich zudosiert wird. Sofern vom Alkoholat und/oder Keton nur Teilmengen vorgelegt werden, muß die portionsweise oder kontinuierliche Zugabe der Restmengen derart erfolgen, daß diese beiden Edukte gegenüber dem Carbonsäureester stets in einem stöchiometrischen Überschuß vorliegen. Die Einspeisung des Alkalialkoholates kann dabei in Form einer Suspension in dem inerten Lösemittel erfolgen. Auch Ester und Keton können als Mischungen untereinander und/oder mit dem Lösemittel zudosiert werden.

Ein besonderer Vorzug des hier beschriebenen Verfahrens ist es, dieses Vermischen den apparativen Gegebenheiten anzupassen und beispielsweise bei entsprechender Wärmeabführung, wie zum Beispiel in doppelmanteligen Metallreaktoren, zügig durchzuführen. Daneben besteht die Möglichkeit, die Einsatzstoffe über einen bestimmten Zeitraum zu dosieren, was zum Beispiel aus Sicherheitsgründen bei schlechter Wärmeableitung, wie etwa im Emailreaktor, von Vorteil sein kann, um Nebenreaktionen und somit Ausbeuteverluste durch Überhitzen des Reaktionsgemisches zu unterbinden.

Im Falle der Dosierung wird erfindungsgemäß vorzugsweise der Carbonsäureester in bis zu 4 Stunden, bevorzugt in 1 bis 2 Stunden, dosiert. Längere Zeiten sind prinzipiell möglich, bringen jedoch keinen entscheidenden Vorteil.

Für ein industriell genutztes Verfahren können zusätzliche Freiheitsgrade von Vorteil sein, die beim erfindungsgemäßen Verfahren durch die Möglichkeit der Verwendung von Ester- und Alkoholatgemischen mit untereinander unterschiedlichen Alkoxyresten erzielt werden. So sind zum Beispiel gleichzeitig Essigsäuremethyl- und -ethylester und z. B. Natrium- und/oder Kaliummmethylat und -ethylat einsetzbar. Die bei der Umsetzung freiwerdende Energie kann auch zum Aufheizen auf Reaktionstemperatur genutzt werden.

Unter wirtschaftlichen Aspekten kommt dem vorliegenden Verfahren besondere Bedeutung zu, da es sich durch besonders kurze Reaktionszeiten von 1 bis 4 Stunden und den damit verbundenen hohen Raum-Zeit-Ausbeuten von bekannten Verfahren abhebt.

Im Rahmen dieser Erfindung konnte gezeigt werden, daß Cyclopropylalkan-1,3-dione durch Reaktion von Cyclopropylalkylketonen mit einem Carbonsäureester in Ausbeuten von > 90 % d. Th. erhalten werden.

Die folgenden Beispiele sollen die Erfindung erläutern.

### Vergleichsbeispiel A:

51,9 g (0,29 mol) einer 39 %igen Kaliummethylatlösung in Methanol werden mit 8,4 g (0,1 mol) CPMK versetzt und bei 30 °C mit 17,6 g (0,2 mol) Essigsäureethylester innerhalb von 30 Minuten versetzt. Nach 24 h zeigte sich noch kein Umsatz an CPMK.

### Beispiel 1: Darstellung von 1-Cyclopropylbutan-1,3-dion

In 75 ml Methyl-tert.-butylether suspendiert man 14,2 g (0,2 mol) Kaliummethylat, fügt 8,4 g (0,1 mol) CPMK hinzu und dosiert innerhalb von 45 Minuten 17,6 g (0,2 mol) Essigsäureethylester hinzu. Nach 3 h bei 30 °C wird mit wäßriger Salzsäure aufgearbeitet und die Leichtsieder nach Extraktion abdestilliert. Rohausbeute: 12,18 g, entspr. 97,4 % d. Th., Gehalt 98,5 Gew.-%.

### Beispiel 2: Darstellung von 1-Cyclopropylbutan-1,3-dion

Die Herstellung erfolgt analog zu Beispiel 1, jedoch unter Zusatz verschiedener Alkohole zum Reaktionsgemisch. Die Mengen in ml pro mol Alkoholat und die Ausbeuten gehen aus Tabelle 1 hervor.

**Tabelle 1**

| Alkohol | Menge [ml/mol] | Ausbeute [% d. Th.] |
|---|---|---|
| tert.-Butanol | 50 | 84 |
| Polyethylenglykol | 50 | 77 |
| 2-Phenoxyethanol | 25 | 84 |
| Methanol | 12,5 | 80 |
| Ethylenglykol | 25 | 60 |

### Beispiel 3: Darstellung von 1-Cyclopropyl-4-phenylbutan-1,3-dion

Analog zu Beispiel 1 wird Phenylessigsäureethylester mit CPMK umgesetzt. Ausbeute: 80 % d. Th.

## Patentansprüche

1. Verfahren zur Herstellung von 1-Cyclopropylalkan-1,3-dionen der Formel IV aus einem Cyclopropylketon der Formel III, einem Alkalialkoholat der Formel II und einem Carbonsäureester der Formel I nach dem Schema 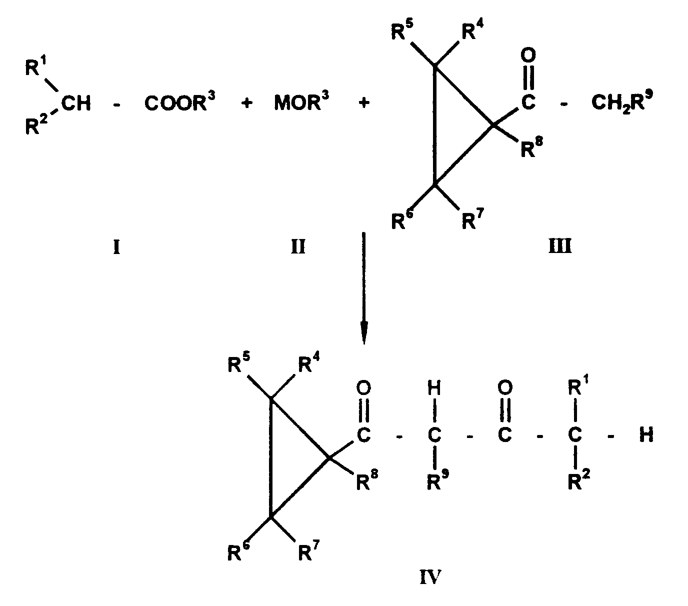 wobei
R¹, R² und R⁴ bis R⁸ gleiche oder unterschiedliche Bedeutung haben und für Wasserstoff oder Alkyl mit 1 bis 6 C-Atomen oder für einen ggf. substituierten Arylrest mit 6 bis 10 C-Atomen steht,
R³ eine Alkylgruppe mit 1 bis 10 C-Atomen ist,
M ein Alkalimetall bedeutet und
R⁹ Wasserstoff oder ein Alkylrest mit 1 bis 20 C-Atomen sein kann,
dadurch gekennzeichnet,
daß die Umsetzung, bei der der Carbonsäureester nicht vor dem Cyclopropylketon mit dem Alkalialkoholat in Kontakt gebracht wird, bei einer Temperatur unterhalb von 50 °C in einem Lösemittel, das alkoholfrei ist oder bis zu 0,7 mol Alkohol pro mol Alkalialkoholat enthält, durchgeführt wird.

2. Verfahren nach Anspruch 1,
dadurch gekennzeichnet,
daß, bezogen auf 1 mol Cyclopropylketon, 1 bis 5 mol, vorzugsweise 1 bis 2 mol Alkalialkoholat und 1 bis 9 mol, vorzugsweise 1 bis 2 mol Carbonsäureester zur Synthese eingebracht werden.

3. Verfahren nach Anspruch 2,
dadurch gekennzeichnet,
daß als Alkalialkoholat Natriummethylat oder -ethylat oder Kaliummethylat oder -ethylat eingesetzt wird.

4. Verfahren nach Anspruch 1,
dadurch gekennzeichnet,
daß die Reaktion im Temperaturbereich zwischen -20 und 50 °C, vorzugsweise bei 0 bis 40 °C, durchgeführt wird.

5. Verfahren nach Anspruch 1,
dadurch gekennzeichnet,
daß R¹, R² sowie R⁴ bis R⁹ für Wasserstoff stehen und das dann resultierende 1-Cyclopropylbutan-1,3-dion durch Acylierung von Cyclopropylmethylketon mit Essigsäuremethylester oder -ethylester und Alkalialkoholat gewonnen wird.

6. Verfahren nach Anspruch 5,
dadurch gekennzeichnet,
daß das Alkalialkoholat zunächst mit dem Cyclopropylmethylketon und dann mit dem Essigsäureester in Kontakt gebracht wird.

## Claims

1. A process for preparing 1-cyclopropylalkane-1,3-diones of the formula IV from a cyclopropyl ketone of the formula III, an alkali metal alcoholate of the formula II and a carboxylic ester of the formula I in accordance with the scheme 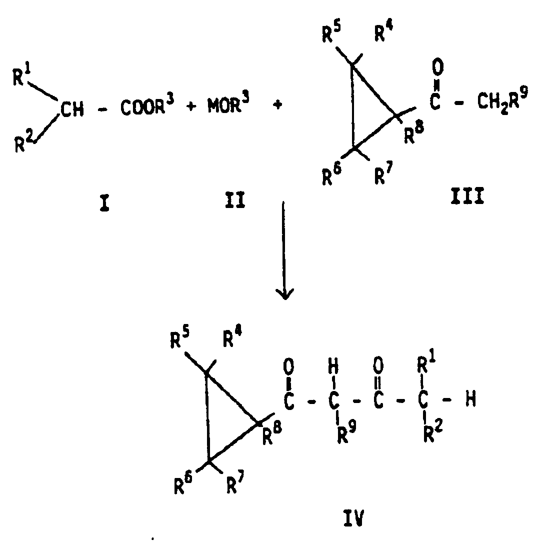 where
R¹, R² and R⁴ to R⁸ have the same or different meanings and represent hydrogen or alkyl having from 1 to 6 C atoms or an optionally substituted aryl radical having from 6 to 10 C atoms,
R³ is an alkyl group having from 1 to 10 C atoms,
M denotes an alkali metal, and
R⁹ can be hydrogen or an alkyl radical having from 1 to 20 C atoms,
characterized in that
the reaction, in which the carboxylic ester is not brought into contact with the alkali metal alcoholate before the cyclopropyl ketone, is carried out at a temperature of less than 50°C in a solvent which is free of alcohol or contains up to 0.7 mol of alcohol per mole of alkali metal alcoholate.

2. A process according to claim 1,
characterized in that,
based on 1 mol of cyclopropyl ketone, from 1 to 5 mol, preferably from 1 to 2 mol, of alkali metal alcoholate and from 1 to 9 mol, preferably from 1 to 2 mol, of carboxylic ester are introduced for the synthesis.

3. A process according to claim 2,
characterized in that,
sodium methoxide or ethoxide or potassium methoxide or ethoxide is employed as the alkali metal alcoholate.

4. A process according to claim 1,
characterized in that,
the reaction is carried out in a temperature range of from -20 to 50°C, preferably at from 0 to 40°C.

5. A process according to claim 1,
characterized in that,
R¹, R² and R⁴ to R⁹ represent hydrogen and the 1-cyclopropylbutane-1,3-dione which then results is obtained by the acylation of cyclopropyl methyl ketone using methyl acetate or ethyl acetate and alkali metal alcoholate.

6. A process according to claim 5,
characterized in that,
the alkali metal alcoholate is initially brought into contact with the cyclopropyl methyl ketone and then with the acetic ester.

## Revendications

1. Procédé de fabrication de 1-cyclopropyl-alkane 1,3-dione de formule IV à partir d'une cyclopropylcétone de formule III, d'un alcoolate de métal alcalin de formule II et d'un ester d'acide carboxylique de formule I selon le schéma : 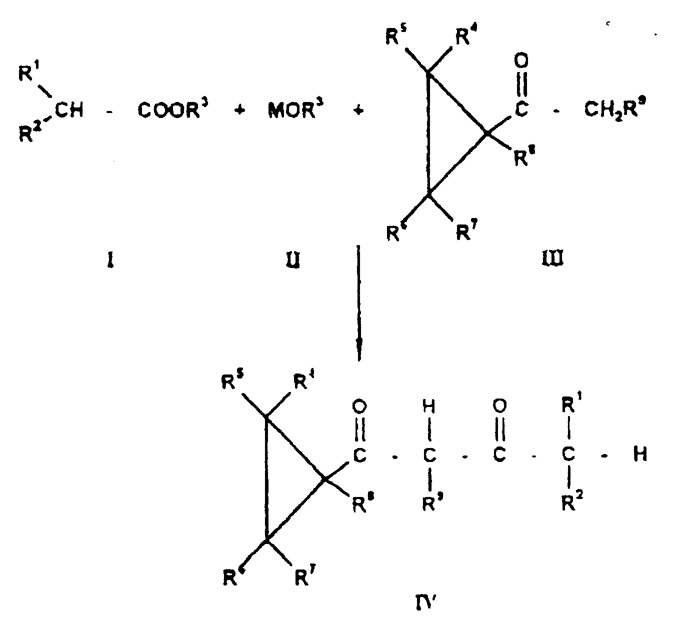 dans lesquelles
R¹, R² et R⁴ à R⁹ ont une signification identique ou différente, et représentent de l'hydrogène ou un radical alkyle ayant de 1 à 6 atomes de carbone ou un radical aryle éventuellement substitué ayant de 6 à 10 atomes de carbone,
R³ est un radical alkyle ayant de 1 à 10 atomes de carbone,
M signifie un métal alcalin, et
R⁷ peut être de l'hydrogène ou un radical alkyle ayant de 1 à 20 atomes de carbone,
caractérisé en ce qu'
on effectue la réaction dans laquelle l'ester d'acide carboxylique n'est pas mis en contact avec l'alcoolate de métal alcalin avant la cyclopropylcétone, à une température en dessous de 50°C, dans un solvant qui est exempt d'alcool ou qui renferme jusqu'à 0,7 mol d'alcool par mol d'alcoolate de métal alcalin.

2. Procédé selon la revendication 1,
caractérisé en ce que
rapporté à une mole de cyclopropylcétone, on introduit dans la synthèse de 1 à 5 moles, de préférence de 1 à 2 mole, d'alcoolate de métal alcalin et de 1 à 9 mole, de préférence 1 à 2 moles d'ester d'acide carboxylique.

3. Procédé selon la revendication 2,
caractérisé en ce qu'
on met en oeuvre comme alcoolate de métal alcalin, le méthylate ou l'éthylate de sodium ou le méthylate ou l'éthylate de potassium.

4. Procédé selon la revendication 1,
caractérisé en ce qu'
on effectue la réaction dans une zone de températures comprise entre -20 et 50°C, de préférence à 0 à 40°C.

5. Procédé selon la revendication 1,
caractérisé en ce que
R¹, R² ainsi que R⁴ à R⁹ représentent de l'hydrogène et que la 1-cyclopropylbutane 1,3-dione qui en résulte donc est extraite par acylation de la cyclopropylméthylcétone avec l'ester méthylique ou l'ester éthylique d'acide acétique et un alcoolate de métal alcalin.

6. Procédé selon la revendication 5,
caractérisé en ce que
l'alcoolate de métal alcalin est amené en contact en premier lieu avec la cyclopropylméthylcétone et ensuite avec l'ester d'acide acétique.
